(19) Europäisches Patentamt
European Patent Office
Office européen des brevets

(11) Veröffentlichungsnummer: **0 299 107 B1**

(12) **EUROPÄISCHE PATENTSCHRIFT**

(45) Veröffentlichungstag der Patentschrift: **06.05.92**

(51) Int. Cl.5: **C07C 69/757**, C07C 67/56

(21) Anmeldenummer: **87110394.1**

(22) Anmeldetag: **17.07.87**

(54) **Verfahren zur Abtrennung und Gewinnung von Dicaffeoyl-chinasäuren.**

(43) Veröffentlichungstag der Anmeldung:
**18.01.89 Patentblatt 89/03**

(45) Bekanntmachung des Hinweises auf die Patenterteilung:
**06.05.92 Patentblatt 92/19**

(84) Benannte Vertragsstaaten:
**AT BE CH DE ES FR GB GR IT LI LU NL SE**

(56) Entgegenhaltungen:
**DE-A- 3 603 574**

(73) Patentinhaber: **ERGO Forschungsgesellschaft mbH**
**Luruper Chaussee 145**
**W-2000 Hamburg 50(DE)**

(72) Erfinder: **Kopsch, Reiner**
**Möwenring 7d**
**W-2000 Schenefeld(DE)**
Erfinder: **Gösswein, Claus F., Dr.**
**Ellernbrook 20**
**W-2110 Buchholz 5(DE)**
Erfinder: **Lutz, Henning, Dr.**
**Hagenwisch 2a**
**W-2083 Halstenbek(DE)**
Erfinder: **Ball, Michael, Dr.**
**Hagenwisch 2c**
**W-2083 Halstenbek(DE)**
Erfinder: **Hubert, Peter**
**Hasenkamp 21**
**W-2150 Buxtehude(DE)**

(74) Vertreter: **UEXKÜLL & STOLBERG Patentanwälte**
**Beselerstrasse 4**
**W-2000 Hamburg 52(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 299 107 B1

Rank Xerox (UK) Business Services
(3.08/2.18/2.0)

## Beschreibung

Die Erfindung betrifft ein einfaches und wirtschaftliches Verfahren zur Abtrennung und Gewinnung von Dicaffeoylchinasäuren durch Extraktion von geeigneten pflanzlichen Rohstoffen und Aufarbeitung des Extrakts, bei dem gegebenenfalls zusätzlich die Monocaffeoylchinasäuren gewonnen werden können.

Mono- und Dicaffeoylchinasäuren kommen in zahlreichen Pflanzen vor, wobei jedoch im allgemeinen der Gehalt an Dicaffeoylchinasäuren im Vergleich zu den Monosäuren sehr gering ist.

Erfindungsgemäß werden mit dem Begriff "Dicaffeoylchinasäuren" - nachfolgend als "DiC" bezeichnet - die 3,4-, 3,5-und 4,5-Dicaffeoylchinasäuren und mit dem Begriff "Monocaffeoylchinasäuren" - nachfolgend als "MonoC" bezeichnet - die 3-, 4-, und 5-Monocaffeoylsäuren sowie deren Salze gemäß den nachfolgend wiedergegebenen Formeln bezeichnet:

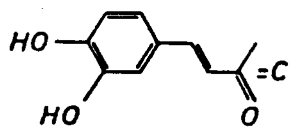

Caffeoyl-

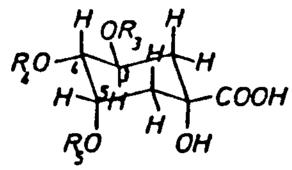

Chinasäure

| $R_3R_4R_5$ | |
|---|---|
| C H H | 3-O-Caffeoylchinasäure |
| H C H | 4-O-Caffeoylchinasäure |
| H H C | 5-O-Caffeoylchinasäure |
| C C H | 3,4-Dicaffeoylchinasäure |
| C H C | 3,5-Dicaffeoylchinasäure |
| H C C | 4,5-Dicaffeoylchinasäure |

Die MonoC und DiC wurden im früheren Sprachgebrauch häufig mit dem Sammelbegriff "Chlorogensäuren" benannt.

K. Gorter, LiebigsAnn. **358**, 327-348 (1908), K. Freudenberg, Ber. **53**, 232-239 (1920) sowie W. Plücker und W. Keilholz, Z. Lebensmittelunters. u. Forsch. **66**, 200-238 (1933) haben Verfahren zur Gewinnung von Chlorogensäure beschrieben. Bei allen bekannten Verfahren ist der erste Schritt die Gewinnung eines Kalium-Coffein-Chlorogenatkomplexes. Dieser wird nach unterschiedlichen Methoden gereinigt, z.B. durch mehrmaliges Umkristallisieren aus Ethanol/Wasser oder durch Fällen mit Bleiacetat. Das Abtrennen des Coffeins aus dem gereinigten Komplex kann beispielsweise durch Extraktion der wässrigen Lösung mit Chloroform erfolgen. Die Chlorogensäure wird anschließend durch Zusatz von Schwefelsäure gefällt und durch Umkristallisieren aus Wasser weiter gereinigt. Die Ausbeute an Chlorogensäure wird mit 1%, bezogen auf die eingesetzte Rohkaffeemenge, angegeben, was bezogen auf die im Rohkaffee enthaltene Menge an Chlorogensäure einer Ausbeute von ca. 20% entspricht.

Andere Verfahren zur Gewinnung von Chlorogensäure beruhen auf einer Modifikation oder Kombination der früheren Verfahren. So beschreibt beispielsweise U. Fiedler, Arzneimittelforschung **4**, 41-45 (1954) ein Verfahren, welches eine Kombination der Methoden von Freudenberg sowie von Plücker und Keilholz darstellt. Danach werden grüne Kaffeebohnen getrocknet und zerkleinert und zunächst mit Petrolether und anschließend mit heißem Wasser extrahiert, bis die Extrakte keine Chlorogensäure mehr enthalten. Die vereinigten Auszüge werden eingeengt und mit Bariumacetat gefällt. Anschließend wird das Filtrat mit Schwefelsäure genau neutralisiert, wobei gleichzeitig das überschüssige Barium entfernt wird. Aus dem neutralen Filtrat wird mit Hilfe von Bleiacetat die Chlorogensäure als Komplex abgeschieden, welcher mit heißem Wasser gewaschen und anschließend nach Aufschlämmen in heißem Wasser mit Schwefelwasserstoff zersetzt wird. Aus dem eingeengten Filtrat scheidet sich nach zwei- bis dreitägigem Stehen im Kühlschrank der Kalium-Coffein-Chlorogenatkomplex ab. Aus dem Komplex wird das Coffein mit Chloroform entfernt und schließlich durch schwaches Ansäuern die freie Chlorogensäure erhalten.

In Chemical Abstracts **73**, 32171E (1970) wird ein Verfahren wiedergegeben, nach dem die isomeren Chlorogensäuren durch Säulenchromatographie an Kieselsäuren und Elution mit einem Chloroform-Butanolgradienten eluiert werden. In Chemical Abstract **89**, 88972D (1978) ist die Extraktion von Chlorogensäure aus einem wässrigen Produkt grüner Kaffeebohnen durch Anionaustauscher wie Dowex 44, Amberlite IRA 410 oder IRA 47 und Dowex 11 beschrieben.

Es ist deutlich, daß die vorbekannten Verfahren recht umständlich sind und keine hohen Ausbeuten liefern können. Spezielle Verfahren zur Herstellung von DiC sind im Stand der Technik nicht beschrieben und soweit diese im Handel überhaupt erhältlich sind, liegt ihr Preis extrem hoch.

Der Erfindung liegt daher die Aufgabe zugrunde, ein einfaches und wirtschaftliches Verfahren zur Abtrennung und Gewinnung von freien DiC oder von Salzen derselben zu entwickeln. Ferner soll ein Verfahren geschaffen werden, mit dem die MonoC und DiC gleichzeitig gewonnen werden können.

Ein einfaches und wirtschaftliches Verfahren zur Herstellung von MonoC ist bereits in der DE-PS 36 03 574 sowie in der Anmeldung PCT/EP87/00049 beschrieben, bei dem man die MonoC mit Hilfe der Gelpermeations-Chromatographie an einem Molekularsieb aus einem vernetzten modifizierten Polysaccharid und insbesondere einem vernetzten Dextran aus dem Extrakt abtrennt und die MonoC und/oder den von MonoC befreiten Extrakt gewinnt.

Die Gelpermeations-Chromatographie an vernetzten modifizierten Polysacchariden dient im allgemeinen der Auftrennung von Substanzgemischen nach der Molekülgröße, d.h. die Moleküle erscheinen im Eluat in der Reihenfolge abnehmender Molekülgröße. Bei einer derartigen Auftrennung eines wässrigen, MonoC enthaltenden pflanzenextraktes war demgemäß zu erwarten, daß die MonoC etwa in der Mitte des Elutionsspektrums und gemeinsam mit einer Reihe von Substanzen mit gleichem oder ähnlichem Molekulargewicht bzw. Molekülgröße erscheinen würden. Überraschenderweise hat es sich jedoch gezeigt, daß die MonoC sich auf einem Molekularsieb aus insbesondere Dextrangel nicht ihrem Molekulargewicht entsprechend verhalten. Gemäß der DE-PS 36 03 547.4 und der Anmeldung PCT/EP87/00049 wurde nämlich festgestellt, daß vernetzte modifizierte Polysaccharide und insbesondere Dextrane ein selektives Rückhaltevermögen für die MonoC aufweisen, d.h., die Säuren werden wesentlich länger festgehalten, als für deren Molekülgröße zu erwarten gewesen wäre. Die Gesamtmenge der in dem Pflanzenextrakt enthaltenen Substanzen mit größerem und kleinerem Molekulargewicht als die MonoC verläßt in einer breiten Fraktion die Trennsäule und die MonoC werden selektiv zurückgehalten. Sie sind durch weiteres Eluieren in relativ reinen und sauber abgetrennten Fraktionen erhältlich.

Überraschenderweise hat es sich erfindungsgemäß gezeigt, daß die 3,4- , 3,5- und 4,5-Isomere der Dicaffeoylchinasäure (DiC) das für die MonoC beschriebene selektive Bindungsvermögen an vernetzte Dextrane in sehr viel stärkerem Ausmaß besitzen als die Monosäuren. Es ist demgemäß eine gelchromatographische Abtrennung der DiC sowohl von unerwünschten Begleitsubstanzen als auch von den MonoC möglich, da erstere das Trennmaterial zu einem sehr viel späteren Zeitpunkt verlassen als die übrigen Bestandteile des Extraktes.

Ein besonders geeignetes Material zur Auftrennung sind Gele von vernetzten Dextranen, wie sie beispielsweise unter der Bezeichnung SEPHADEX® vertrieben werden. Erfindungsgemäß zeigte es sich ferner, daß die Trennschärfe mit steigendem Vernetzungsgrad des verwendeten Dextrangels ansteigt; d.h., bei vorgegebener Säulenlänge wird mit höher vernetztem Dextrangel eine schärfere Trennung erreicht als bei der Verwendung eines Gels mit niedrigerem Vernetzungsgrad. Als Hinweis auf den Vernetzungsgrad eines Gels kann dessen Quellfähigkeit dienen. Letztere sinkt mit steigendem Vernetzungsgrad. So werden beispielsweise zur Herstellung von 100 ml gequollenem gel 10 g Trockenmaterial des niedrig vernetzten SELPHADEX® G 50 benötigt, während zur Herstellung des gleichen Gelvolumens aus den höher vernetzten SEPHADEX® G 25 und SEPHADEX® G 10 20 bzw. 40 g Trockenmaterial erforderlich sind.

Die geeignete Arbeitstemperatur für die Auftrennung liegt im Bereich von etwa 10 - 90 °C, wobei normalerweise eine Temperatur von 50 °C - 70 °C bevorzugt ist. Üblicherweise werden wässrige Pflanzenextrakte für die Auftrennung verwendet. Das Elutionsmedium kann somit ausschließlich aus Wasser, aber auch aus einem Gemisch von Wasser mit beispielsweise Alkoholen bestehen. Durch die Wahl des Lösungsmittelgemisches läßt sich die jeweils gewünschte Polarität des Lösungsmittelsystems beeinflussen.

Erfindungsgemäß umfaßt der Begriff Dicaffeoylchinasäuren (DiC) sowohl die freien Säuren als auch deren Salze. Bei pH-Werten, wie sie sich in wässrigen Pflanzenextrakten einstellen, dürften die DiC überwiegend in Salzform vorliegen. So beträgt der pH-Wert eines Rohkaffee-Extrakts etwa 5,5.

Sollen im wesentlichen freie DiC nach dem erfindungsgemäßen Verfahren hergestellt werden, so muß der pH-Wert des Extrakts entsprechend gesenkt werden. Es wurde zudem festgestellt, daß das Rückhaltevermögen von vernetzten Dextranen für DiC bei Trennungen im sauren Bereich, beispielsweise bei pH-Werten von etwa 1,5 bis 3 noch stärker ausgeprägt ist, als bei höheren pH-Werten. Es ist dabei nicht wesentlich, welche Säure zur Erniedrigung des pH-Wertes zugesetzt wird. Mineralsäuren wie Salzsäure und

Schwefelsäure haben sich als besonders geeignet erwiesen. Bei bestimmten Aufgabenstellungen kann es zudem günstiger sein, den erforderlichen sauren pH-Wert ohne Säurezusatz einzustellen. Dies kann dadurch erreicht werden, daß der Extraktlösung mit einem Ionenaustauscher die Kationen entzogen werden.

Aufgrund des besonders ausgeprägten Rückhaltevermögens von vernetzten Dextranen für DiC können insbesondere im sauren Bereich schwächer vernetzte Dextrangele wie beispielsweise SEPHADEX® G 50 verwendet werden. Durch den Einsatz von verhältnismäßig langen Säulen mit einer schwach vernetzten Gelfüllung (beispielsweise 25 cm Länge, 2,5 cm Durchmesser, SEPHADEXR® G 50) kann zudem eine saubere Auftrennung der DiC in die einzelnen Isomere erreicht werden.

Sollen die DiC in Salzform nach dem erfindungsgemäßen Verfahren abgetrennt und gewonnen werden, so kann bei dem natürlichen pH-Wert des wässrigen Pflanzenextraktes gearbeitet werden. Dies hat den Vorteil, daß die von dem DiC-Salz befreiten Extrakte unverändert bleiben und im Lebensmittelbereich weiterverwendet werden können.

Als Ausgangsmaterial für die Gewinnung der DiC eignen sich pflanzliche Rohstoffe, welche diese Säuren in ausreichender Konzentration enthalten. Die DiC kommen im allgemeinen neben den MonoC vor, sind jedoch in erheblich geringeren Mengen als letztere vorhanden. Das am besten geeignete Ausgangsmaterial zur Gewinnung von DiC ist Rohkaffee. Der Gehalt verschiedener Rohkaffeesorten an DiC wurde quantitativ mittels Hochdruckflüssigkeitschromatographie (HPLC) analysiert. Die Ergebnisse sind in der nachfolgenden Tabelle wiedergegeben:

Tabelle 1

| Rohkaffee | 4,5-DiC % Tr. | 3,5-DiC % Tr. | 3,4-DiC % Tr. | Summe-DiC % Tr. |
|---|---|---|---|---|
| Columbia | 0,18 | 0,28 | 0,39 | 0,85 |
| Kenia | 0,15 | 0,23 | 0,32 | 0,70 |
| Santos | 0,17 | 0,25 | 0,32 | 0,74 |
| Robusta (Thai.) | 0,41 | 0,39 | 0,57 | 1,37 |
| Robusta (Brasil) | 0,50 | 0,48 | 0,75 | 1,73 |

Wie in Figur 1 dargestellt, können die DiC aufgrund des ausgeprägten Rückhaltevermögens von vernetzten Dextranen an sehr kurzen Säulen auf schnelle und einfache Weise von den gesamten, in dem Pflanzenextrakt enthaltenen Begleitstoffen abgetrennt werden. Derartige Säulen reichen nicht aus, um die MonoC gemäß der DE-PS 36 03 574.2 sowie der Anmeldung PCT/EP87/00049 von den Begleitstoffen abzutrennen.

Gemäß einer besonders bevorzugten Ausführungsform der vorliegenden Erfindung wird der wässrige Pflanzenextrakt und insbesondere der Rohkaffee-Extrakt zur Gewinnung sowohl der MonoC als auch der DiC aufgearbeitet.

Zu diesem Zweck wird mit zwei unterschiedlichen Säulen gearbeitet, wobei die erste Säule lediglich der Abtrennung der DiC von den gesamten übrigen Begleitmaterialien dient. Diese Säule kann beispielsweise eine Länge von 6 cm und ein Gelvolumen von 3 l aufweisen und als Trennmaterial SEPHA-DEX® G 15 enthalten. Nach Aufbringen des Rohkaffeeextraktes wird mit Wasser eluiert und das zu Beginn austretende Eluat, welches ein Gemisch aus Begleitstoffen und MonoC darstellt, wird direkt auf eine zweite, erheblich längere Trennsäule geleitet, auf welcher die MonoC von den Begleitstoffen abgetrennt und gegebenenfalls in ihre Isomeren aufgetrennt werden.

Wie in der PCT/EP87/00049 beschrieben (vgl. S. 7 ff), ist die Trennschärfe bei vorgegebener Säulenlänge sowohl eine Funktion des pH-Wertes des aufzutrennenden Pflanzenextraktes als auch des Vernetzungsgrades des verwendeten Dextrangels. Dort wurden aus entkoffeiniertem Rohkaffee-Extrakt die MonoC jeweils sowohl ohne Ansäuern des Extraktes bei dem natürlichen pH-Wert von 5,6 als auch nach Ansäuern bei pH 2,5 an den SEPHADEX® G 25, G 15 und G 10 abgetrennt, wobei G 25 den niedrigsten und G 10 den höchsten Vernetzungsgrad aufweist. Es wurde ansonsten unter konstanten Bedingungen gearbeitet, und es zeigte sich, daß bei einfach pH-Wert von 2,5 bereits mit dem niedrig vernetzten SEPHADEX® G 25 eine deutliche Abtrennung der MonoC erhalten wurde, während bei pH 5,6 der Peak der Begleitstoffe noch deutlich mit demjenigen der MonoC überlappte. Demgegenüber wurde bei Verwendung des höher vernetzten Gels G 15 auch bei dem nicht angesäuerten Extrakt eine nahezu vollständige Abtrennung der Chlorogensäure von den übrigen Bestandteilen erreicht. Bei Verwendung von SEPHADEX® G 10 trat auch bei dem nicht angesäuerten Extrakt durch die beginnende Differenzierung der einzelnen Isomeren eine verbreiterte Basis des Peaks der MonoC auf.

Zur Abtrennung der MonoC von den Begleitstoffen wird demgemäß das aus der kurzen Säule austretende Anfangseluat in Abhängigkeit von dem verwendeten pH-Wert auf eine Säule mit Dextrangelen, beispielsweise SEPHADEX®, geleitet, die sowohl bezüglich ihrer Länge als auch bezüglich des verwendeten Vernetzungsgrades ausreicht, um die MonoC gemäß PCT/EP87/00049 von den Begleitstoffen abzutrennen und gegebenenfalls in die einzelnen Isomeren aufzutrennen.

Die Überleitung des Eluats aus der kurzen Säule auf diese Trennanordnung wird unterbrochen, wenn die DiC auszutreten beginnen. Diese werden nunmehr gesondert aufgefangen. Die für die MonoC beschriebene Abhängigkeit der Trennschärfe vom pH-Wert des aufzutrennenden Pflanzenextraktes sowie vom Vernetzungsgrad des verwendeten Dextrangels gilt entsprechend auch für die DiC, mit dem Unterschied, daß bei diesen jeweils bei höherem pH-Wert bzw. niedrigerem Vernetzungsgrad als bei den MonoC eine gute Auftrennung erreicht wird. Ist daher eine weitere Auftrennung der DiC in einzelne Isomeren erwünscht, so kann das Eluat ebenfalls auf eine längere Säule und/oder eine Säule mit höher vernetztem Gel geleitet werden; die geeignete Säulenlänge und der Vernetzungsgrad können durch einfache Vorversuche ermittelt werden.

Die einzelnen Eluat-Fraktionen können durch Hochdruckflüssig-keits-Chromatographie (HPLC) analysiert werden. Auf diese Weise kann für ein bestimmtes Säulenmaterial und eine gegebene Säulenlänge festgestellt werden, welche Eluatfraktionen die gewünschte Säure enthalten und inwieweit der jeweilige Vorlauf abzutrennen ist. Aus den die MonoC bzw. DiC enthaltenden Eluatfraktionen können die Säuren auf übliche Weise, besonders schonend beispielsweise durch Gefriertrocknung isoliert und als Festsubstanz gewonnen werden.

Gegenüber den bislang bekannten Verfahren bietet das erfindungsgemäße eine Reihe von Vorteilen. Das Verfahren ist wesentlich einfacher, da es weniger Verfahrensschritte und nur geringen apparativen Aufwand erfordert. Besonders wesentlich ist, daß die im pflanzlichen Rohstoff enthaltenen DiC gegebenenfalls gleichzeitig mit den MonoC weitgehend vollständig isoliert und gewonnen werden können, wobei das Gesamtverfahren ohne chemische Hilfsmittel wie beispielsweise organische Lösungsmittel durchgeführt werden kann.

Gemäß einer weiteren Ausführungsform der Erfindung werden die Mono- und Dicaffeoylchinasäuren wie oben beschrieben aus dem pflanzlichen Rohextrakt entfernt und der von den Säuren befreite Extrakt als Endprodukt gewonnen. Wie Figur 4 zeigt, kann nach dem erfindungsgemäßen Verfahren ein im wesentlichen vollständig von Mono- und Dicaffeoylsäuren freier Pflanzenextrakt hergestellt werden.

Es besteht ferner die Möglichkeit, das Verfahren semikontinuierlich durchzuführen, da die Trenneigenschaften des Dextrans offensichtlich sehr lange erhalten bleiben. So zeigte z.B. eine mit vernetztem Dextran gefüllte Säule auch nach über 100 Durchgängen unverändert reproduzierbare Eigenschaften. Ein Durchgang besteht jeweils aus

- Auftragen des Extraktes,
- Eluieren der Begleitstoffe, wobei gegebenenfalls die Begleitstoffe zur Isolierung der MonoC auf eine weitere Säule geleitet werden,
- Eluieren von DiC und
- Spülen.

Unmittelbar an das Spülen des Gels kann der nächste Durchgang angeschlossen werden. Bei Verschmutzung der Säule kann die Füllung mit einfachen, im Stand der Technik bekannten Mitteln gereinigt werden. Schwebstoffe, die den Durchlauf verschlechtern, können vor Aufrühren des Gels in Wasser abgeschwemmt werden. Vom Gel festgehaltene Farbstoffe können mit 0,2%iger Natronlauge im Durchlauf entfernt werden, ohne daß dadurch das Trennverhalten beeinflußt wird. Zur näheren Erläuterung der Erfindung sollen die nachfolgenden Beispiele dienen.

Beispiel 1

Gewinnung von Dicaffeoylchinasäure in Salzform aus Rohkaffee

3,0 kg entcoffeinierter Rohkaffee (Herkunftsland Columbien) wurden mit 15 l entmineralisiertem Wasser bei 80 ° C extrahiert. Der erhaltene Extrakt wies einen pH-Wert von 5,6 auf. Die Lösung wurde anschließend auf 1,75 kg eingeengt. Die Extraktkonzentration betrug 23,36 g Trockensubstanz/100 g Flüssigkeit. 15 g dieses Extrakts wurden mit einer Schlauchpumpe auf eine Säule mit 30 ml Gel (SEPHADEX® G 15) gefördert. Die Höhe der Gelschicht betrug 6 cm.

Die Säule wurde mit Wasser bei einer Temperatur von 50 ° C und einer Fließgeschwindigkeit von 300 ml/h eluiert. Das Eluat wurde kontinuierlich durch Leitfähigkeitsmessung überwacht. Der erste Substanzpeak hatte die Säule nach einer Elutionsmenge von 75 g verlassen. Diese Menge wurde abgetrennt und als

Fraktion A bezeichnet. Daran anschließend wurde ein zweiter Peak von der Säule eluiert, welcher 175 g umfaßte. Diese mit "B" bezeichnete Fraktion wurde ebenfalls gesammelt. Die Fraktionen wurden sowohl bezüglich des Trockensubstanz-Gehalts als auch mittels HPLC untersucht, um den Gehalt an Dicaffeoylchinasäuren zu bestimmen.

Die Ergebnisse sind in der Tabelle 2 sowie in den Figuren 1 bis 3 wiedergegeben.

In den Figuren bedeuten:

I = 5-Caffeoylchinasäure
II = 4-Caffeoylchinasäure
III = 3-Caffeoylchinasäure
IV = 4,5-Dicaffeoylchinasäure
V = 3,5-Dicaffeoylchinasäure
VI = 3,4-Dicaffeoylchinasäure

Die für die Peaks angegebenen Zahlen sind Retentionszeiten in Minuten.

Tabelle 2

| | Flüssigkeits-Menge. | | Trockensubstanz | | DiC | DiC |
|---|---|---|---|---|---|---|
| | g | g/100g | g | g/100g | g | % i.Tr. |
| Ausgangsextrakt | 15 | 23,36 | 3,50 | 0,47 | 0,07 | 2,0 |
| Fraktion A | 75 | 4,36 | 3,27 | 0 | 0 | 0 |
| Fraktion B | 175 | 0,07 | 0,12 | 0,04 | 0,07 | 58,3 |

Figur 1 zeigt den Elutionsverlauf des eingesetzten Extrakts aus entcoffeiniertem Columbia-Rohkaffee.

Die HPLC-Analyse wurde mittels Gradientenelution unter den folgenden Bedingungen durchgeführt:

| | |
|---|---|
| Säule | ET 250/8/4 Nucleosil 5C18 HOP (Macherey und Nagel) |
| mobile Phase | Methanol-Essigsäure (2%ig) |
| Flußrate | 0,8 ml/min |
| Detektor | Biotronic-UV-BT 3030 bei 325 nm |
| Integrator | Shimadzu CR1B (Steigerung der Empfindlichkeit nach 25 Minuten) |

Figur 2 zeigt das HPLC-Chromatogramm des eingesetzten Extrakts aus entcoffeiniertem Columbia-Rohkaffee.

Figur 3 zeigt das entsprechende HPLC-Chromatogramm der Fraktion B. Ausweislich des Chromatogramms enthält die Fraktion B nur die Isomeren der DiC.

## Beispiel 2

### Gleichzeitige Gewinnung von Mono- und Dicaffeoylchinasäuren

Der Extrakt aus entcoffeiniertem Rohkaffee gemäß Beispiel 1 wurde in einem aus den Säulen I und II bestehenden System aufgetrennt.

Die Säule I enthielt ein Gelvolumen (SEPHADEX® G 15) von 3,0 l; die Höhe des Gelbettes betrug 6 cm. Die Säule II hatte ein Gelvolumen von 11 l (SEPHADEX® G 15); die Höhe des Gelbettes betrug 22 cm.

Die Säulen wurden mit Wasser bei einer Temperatur von 65 °C und einer Fließgeschwindigkeit von 33 l/h eluiert.

Zu Beginn des Trennvorganges war der Ausgang der Säule I mit dem Eingang der Säule II verbunden. Mit einer Schlauchpumpe wurden zunächst 1,57 kg des aufzutrennenden Extrakts auf die Säule I geleitet und anschließend mit Wasser eluiert. Das austretende Eluat wurde durch Leitfähigkeitsmessung überwacht und solange direkt auf die Säule II geleitet, bis der zweite Peak am Ausgang der Säule I erschien. An diesem Punkt wurde die Verbindung der Säulen getrennt und beide Säulen wurden unabhängig voneinander weiter mit Wasser eluiert. Von der Säule II wurden die Fraktionen 1 und 2 gesammelt, wobei die

Fraktion 1 wie in der DE-PS 36 03 574 und der Anmeldung PCT/EP87/00049 beschrieben die von sämtlichen Chlorogensäuren befreiten Bestandteile des Rohkaffee-Extraktes und die Fraktion 2 die Monocaffeoylchinasäuren enthielt.

Aus der Säule I wurden parallel die Dicaffeoylchinasäuren eluiert und in einer Fraktion gesammelt, die nachfolgend als Fraktion 3 bezeichnet ist.

Die Fraktionen 1, 2 und 3 wurden sowohl hinsichtlich des Trockensubstanzgehalts als auch mit HPLC hinsichtlich der darin enthaltenen Mono- bzw. Dicaffeoylchinasäuren analysiert. Die Ergebnisse sind nachfolgend in Tabelle 3 sowie in den Figuren 3 bis 5 wiedergegeben.

Tabelle 3

| | Flüssigkeits-Menge | Trockensubstanz | | Monocaffeoylchinasäuren | | | Dicaffeoylchinasäuren | | |
|---|---|---|---|---|---|---|---|---|---|
| | | Konz. | Gesamtmenge | Konz. | Gesamt-menge | % in Tr.-Subst. | Konz. | Gesamtmenge | % in Tr.-Subst. |
| | kg | g/100 g | g | g/100 g | g | | g/100 g | g | |
| Ausgangsextrakt | 1,57 | 23,36 | 366,75 | 4,88 | 76,62 | 20,89 | 0,47 | 7,38 | 2,01 |
| Fraktion 1 (von Chlorogensäuren weitestgehend befreit) | 6,49 | 3,53 | 229,10 | 0,01 | 0,65 | 0,28 | 0,002 | 0,13 | 0,06 |
| Fraktion 2 (Monocaffeoyl-chinasäuren) | 9,33 | 1,19 | 111,03 | 0,80 | 74,64 | 67,23 | 0 | 0 | 0 |
| Fraktion 3 (Dicaffeoyl-chinasäuren) | 18,00 | 0,065 | 11,70 | 0,002 | 0,36 | 3,08 | 0,040 | 7,20 | 61,54 |

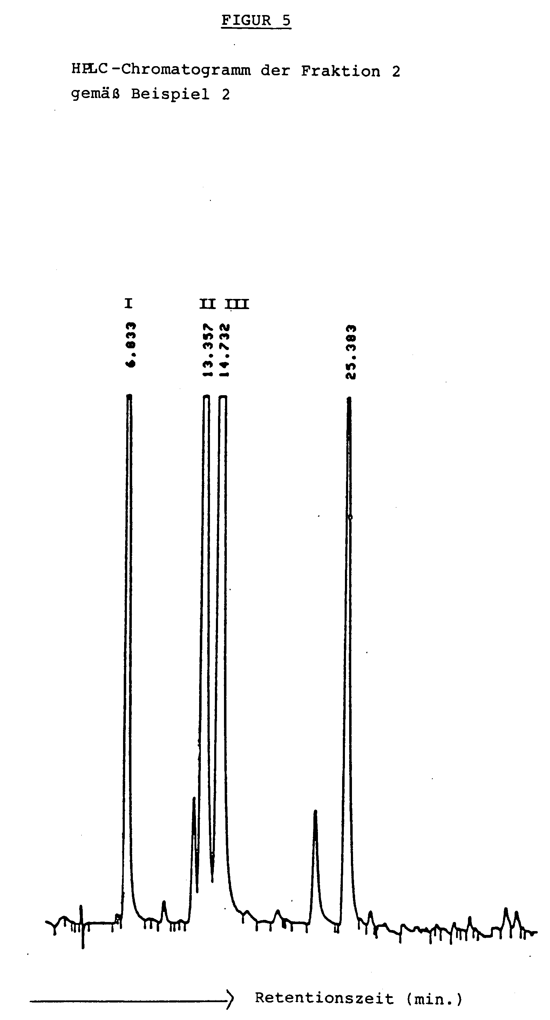

Die Figuren 4 und 5 zeigen die HPLC-Chromatogramme der Fraktionen 1 und 2. Das entsprechende Chromatogramm der Fraktion 3 stimmte mit denjenigen der Fraktion B gemäß Beispiel 1 (vgl. Figur 3) überein.

## Patentansprüche

1. Verfahren zur Abtrennung und/oder Gewinnung von Dicaffeoylchinasäuren gegebenenfalls unter gleichzeitiger Abtrennung und Gewinnung der Monocaffeoylchinasäuren durch Extraktion von pflanzlichen Rohstoffen und Aufarbeitung des gewonnenen Extraktes durch Säulenchromatographie, **dadurch gekennzeichnet,** daß man die Dicaffeoylchinasäuren mit Hilfe der Gelpermeations-Chromatographie an einem vernetzten Dextran von den Begleitstoffen abtrennt und die Dicaffeoylchinasäuren und/oder den von den Dicaffeoylchinasäuren befreiten Extrakt gewinnt und gegebenenfalls die Monocaffeoylchinasäuren aus dem erhaltenen Extrakt abtrennt.

2. Verfahren nach Anspruch 1, **dadurch gekennzeichnet,** daß man zur Gewinnung von im wesentlichen freien Dicaffeoylchinasäuren den wäßrigen Pflanzenextrakt auf einen pH-Wert von etwa 1,5 bis 3,0 einstellt.

3. Verfahren nach den Ansprüchen 1 oder 2, **dadurch gekennzeichnet,** daß man zur Gewinnung der isomeren 3,4, 3,5- und 4,5-Dicaffeoylchinasäuren die Gelpermeations-Chromatographie an mindestens zwei Dextrangelen von unterschiedlichem Vernetzungsgrad durchführt, wobei das erste Gel den niedrigeren Vernetzungsgrad besitzt, anschließend den die Dicaffeoylchinasäuren enthaltenden Teil des Eluats des ersten Gels auf das zweite Gel gibt und das zweite Gel als ausreichend lange Säule einsetzt, aus welcher die isomeren 3,4-, 3,5- und 4,5-Dicaffeoylchinasäuren nacheinander eluiert und die Eluate getrennt aufgefangen werden können.

4. Verfahren nach den Ansprüchen 1 oder 2, **dadurch gekennzeichnet,** daß man zur gleichzeitigen Abtrennung und Gewinnung der Mono- und Dicaffeoylchinasäuren ein System aus mindestens zwei miteinander verbundenen Säulen einsetzt, wobei das von den Dicaffeoylchinasäuren befreite Eluat der ersten Säule direkt auf die zweite Säule geleitet wird und die Verbindung zwischen den Säulen unterbrochen wird, wenn die Dicaffeoylchinasäuren im Eluat der ersten Säule erscheinen und man anschließend die Eluate der ersten und zweiten Säule getrennt auffängt.

5. Verfahren nach Anspruch 4, **dadurch gekennzeichnet,** daß man die getrennt aufgefangenen Eluate der ersten und zweiten Säulen getrennt auf Säulen ausreichender Länge und/oder auf Gele mit ausreichendem Vernetzungsgrad leitet, um auf diese Weise die getrennt aufgefangenen Mono- und Dicaffeoylchinasäuren in ihre jeweiligen Isomere aufzutrennen.

6. Verfahren nach den Ansprüchen 1 bis 5, **dadurch gekennzeichnet,** daß man die Mono- und Dicaffeoylchinasäuren aus dem Extrakt pflanzlicher Rohstoffe abtrennt und den von den Säuren befreiten Extrakt gewinnt.

## Claims

1. Process for the separation and/or preparation of dicaffeoylquinic acids, optionally with simultaneous separation and preparation of the monocaffeoylquinic acids by extraction of vegetable raw materials and working-up of the produced extract by column chromatography, **characterized in that** the dicaffeoylquinic acids are separated from the accompanying substances with the help of gel-permeation chromatography on a cross-linked dextran and the dicaffeoylquinic acids and/or the extract freed of the dicaffeoylquinic acids are prepared and, optionally, the monocaffeoylquinic acids are separated from the obtained extract.

2. Process according to claim 1, **characterized in that,** in order to prepare essentially free dicaffeoylquinic acids, the aqueous plant extract is set to a pH value of ca. 1.5 to 3.0.

3. Process according to claims I or 2, **characterized in that,** in order to prepare the isomeric 3,4-, 3,5- and 4,5-dicaffeoylquinic acids, the gel-permeation chromatography is carried out on at least two dextran gels of different degrees of cross-linking, the first gel having the lower degree of cross-linking, and that

the part of the eluate of the first gel containing the dicaffeoylquinic acids is then added onto the second gel and the second gel is used as a column of adequate length out of which the isomeric 3,4-, 3,5- and 4,5-dicaffeoylquinic acids are eluted in succession and the eluates can be collected separately.

**4.** Process according to claims 1 or 2, **characterized in that** in order to achieve the simultaneous separation and preparation of the mono- and dicaffeoylquinic acids, a system comprising at least two columns connected to each other is used, the eluate which is freed of the dicaffeoylquinic acids being conducted from the first column directly to the second column and the connection between the columns being broken if the dicaffeoylquinic acids appear in the eluate of the first column, and the eluates of the first and second columns are then collected separately.

**5.** Process according to claim 4, **characterized in that** the separately collected eluates of the first and second columns are conducted separately to columns of adequate length and/or onto gels with an adequate degree of cross-linking in order to thus split the separately collected mono- and dicaffeoylquinic acids into their respective isomers.

**6.** Process according to claims 1 to 5, **characterized in that** the mono- and dicaffeoylquinic acids are separated from the extract of vegetable raw materials and the extract freed from the acids is prepared.

## Revendications

**1.** Procédé pour séparer et/ou obtenir des acides dicaféylquiniques, éventuellement avec séparation et obtention simultanée des acides monocaféylquiniques, par extraction de matières premières végétales et traitement de l'extrait obtenu par chromatographie sur colonne, caractérisé en ce qu'on sépare les acides dicaféylquiniques des impuretés associées au moyen de la chromatographie par perméation de gel sur un dextrane réticulé, et on récupère les acides dicaféylquiniques, et ou l'extrait débarrassé des acides dicaféylquiniques et on sépare éventuellement les acides monocaféylquiniques de l'extrait obtenu.

**2.** Procédé selon la revendication 1, caractérisé en ce qu'on ajuste le pH de l'extrait végétal aqueux entre environ 1,5 et 3, pour récupérer principalement des acides dicaféylquiniques libres.

**3.** Procédé selon la revendication 1 ou 2, caractérisé en ce que, pour récupérer les acides isomères 3,4-, 3,5- et 4,5-dicaféylquiniques, on effectue la chromatographie par perméation de gel sur au moins deux gels de dextrane ayant des degrés de réticulation différents, le premier gel possèdant le degré de réticulation le plus bas, on fait ensuite passer la fraction de l'éluat du premier gel, contenant les acides dicaféylquiniques, sur le deuxième gel, et on utilise le deuxième gel comme une colonne de longueur suffisante, à partir de laquelle on peut éluer successivement les acides isomères 3,4-, 3,5- et 4,5-dicaféylquiniques, et recueillir séparèment les éluats.

**4.** Procédé selon la revendication 1 ou 2, caractérisé en ce que, pour séparer et obtenir simultanément les acides mono- et dicaféylquiniques, on utilise un système composé d'au moins deux colonnes reliées l'une à l'autre, l éluat de la première colonne, débarrassé des acides dicaféylquiniques, étant amené directement à la deuxième colonne, et on interrompt la liaison entre les colonnes quand les acides dicaféylquiniques apparaissent dans l'éluat de la première colonne, et on recueille ensuite séparèment les éluats des première et deuxième colonnes.

**5.** Procédé selon la revendication 4, caractérisé en ce qu'on amène séparèment les éluats des première et deuxième colonnes, recueillis séparèment, sur des colonnes de longueur suffisante et/ou sur des gels ayant un degré de réticulation suffisant, pour séparer de cette manière les acides mono- et dicaféylquiniques, recueillis séparèment, en chacun de leurs isomères.

**6.** Procédé selon les revendications 1 à 5, caractérisé en ce qu'on sépare les acides mono- et dicaféylquiniques de l'extrait de matières premières végétales et on obtient l'extrait débarrassé des acides.

FIGUR 1

Abtrennung von Dicaffeoylchinasäure aus entcoffeiniertem Rohkaffee-Extrakt mit SEPHADEX® G 15,
Gelvolumen: 30 ml
Gelhöhe: 6 cm

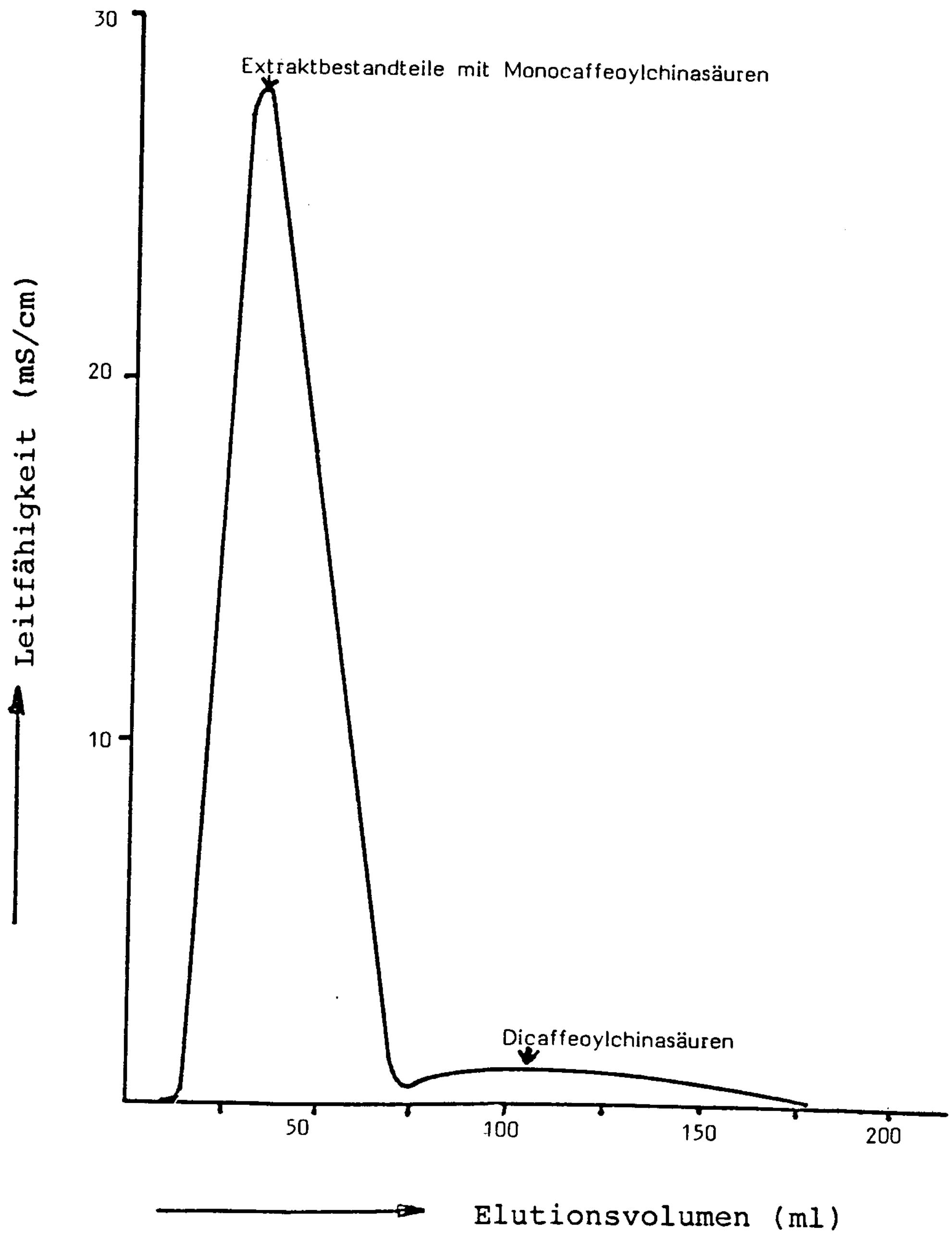

FIGUR 2

HPLC-Chromatogramm eines Extrakts aus entcoffeiniertem Columbia-Rohkaffee

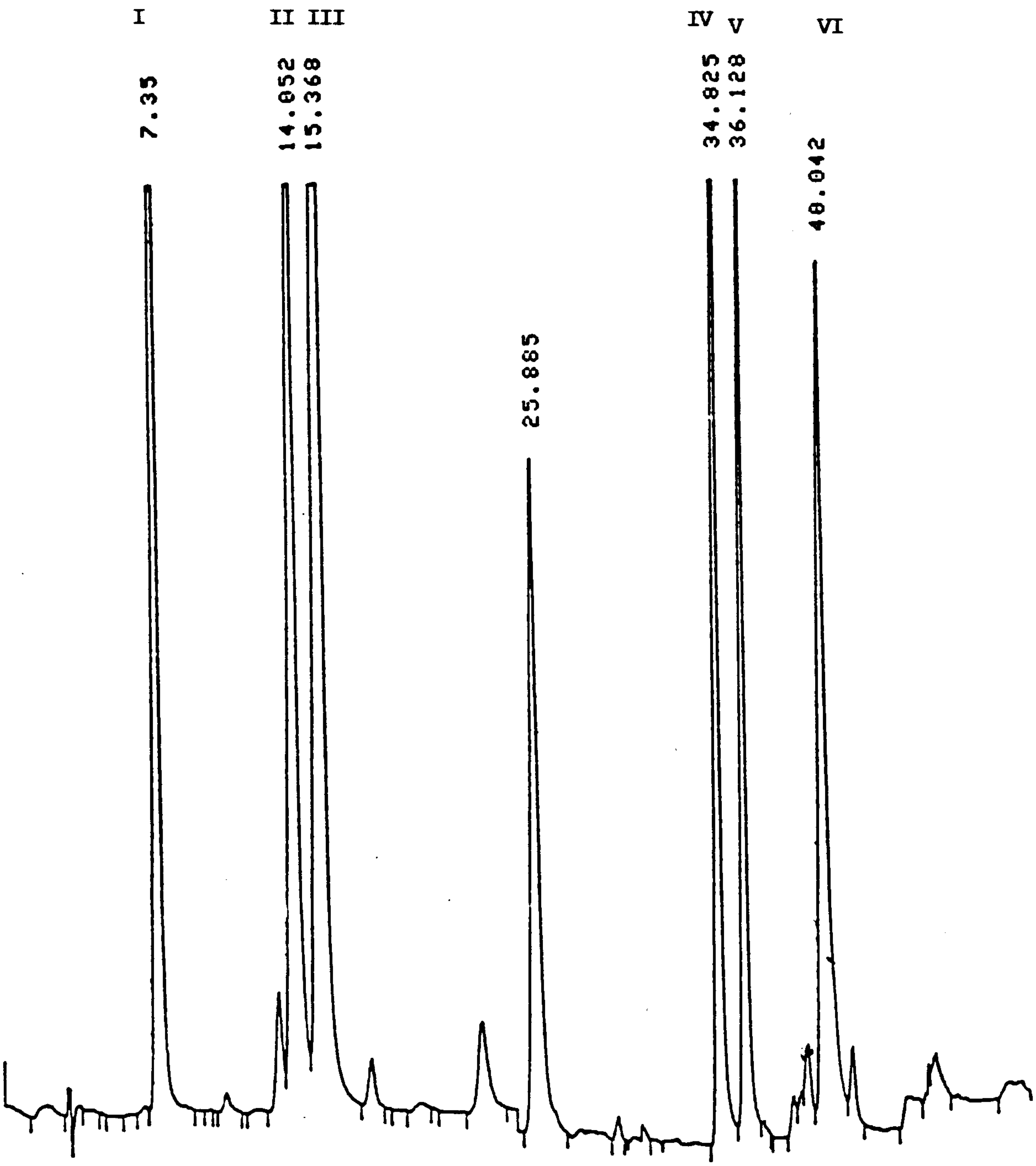


Retentionszeit (min.)

FIGUR 3

HPLC-Chromatogramm der Fraktion B
gemäß Beispiel 1
(entspricht auch Fraktion 3 gemäß Beispiel 2)

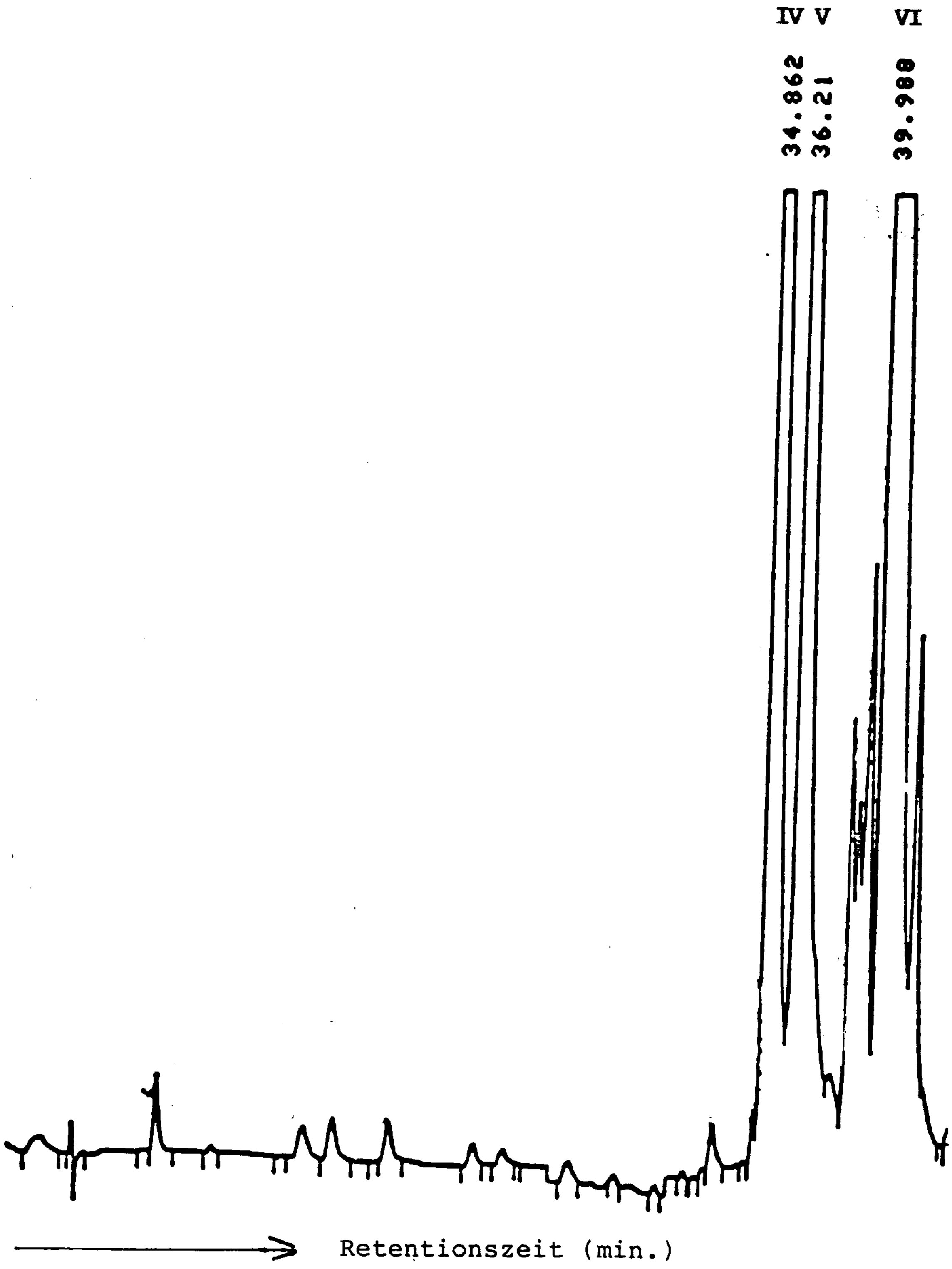

FIGUR 4

HPLC-Chromatogramm der Fraktion 1
gemäß Beispiel 2

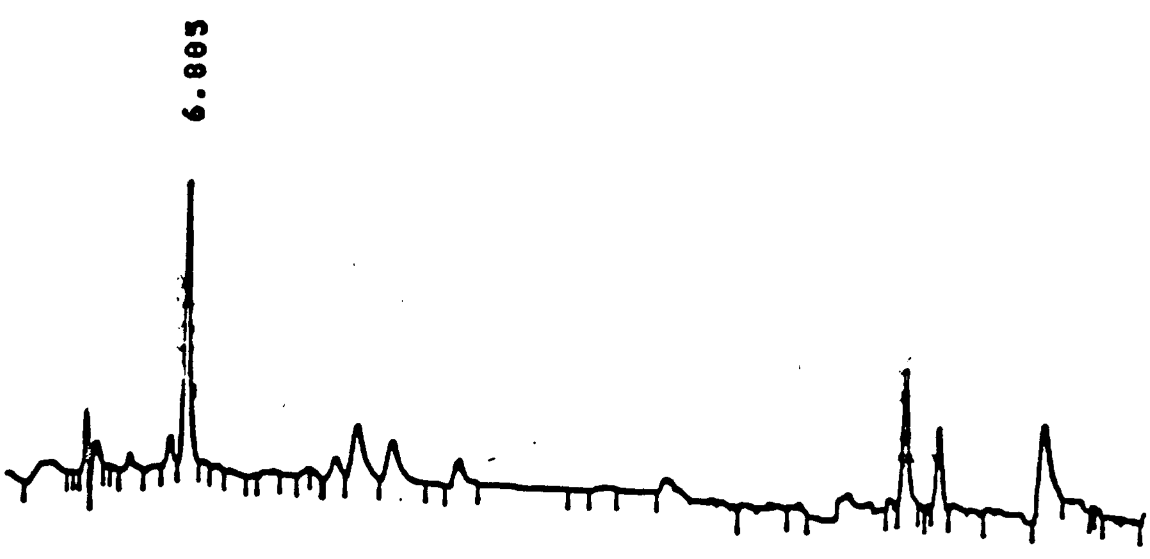


Retentionszeit (min.)

FIGUR 5

HPLC-Chromatogramm der Fraktion 2
gemäß Beispiel 2